(19) Europäisches Patentamt
European Patent Office
Office européen des brevets

(11) Veröffentlichungsnummer: **0 392 354 A1**

(12) # EUROPÄISCHE PATENTANMELDUNG

(21) Anmeldenummer: **90106519.3**

(22) Anmeldetag: **05.04.90**

(51) Int. Cl.⁵: **C07D 207/416, C09J 179/08**

(30) Priorität: **13.04.89 DE 3912081**

(43) Veröffentlichungstag der Anmeldung:
**17.10.90 Patentblatt 90/42**

(84) Benannte Vertragsstaaten:
**CH DE ES FR GB IT LI**

(71) Anmelder: **BASF Aktiengesellschaft**
**Carl-Bosch-Strasse 38**
**D-6700 Ludwigshafen(DE)**

(72) Erfinder: **Dittrich, Uwe, Dr.**
**Pfalzgrafenstrasse 21**
**D-6700 Ludwigshafen(DE)**
Erfinder: **Eisenbarth, Philipp, Dr.**
**Gutleutstrasse 12**
**D-6702 Bad Duerkheim(DE)**
Erfinder: **Kahl, Thomas-Michael, Dr.**
**Germersheimer Strasse 146**
**D-6725 Roemerberg(DE)**

(54) **Härtbare Bismaleinimid-Addukte.**

(57) Die Erfindung betrifft ein Addukt aus einem Bismaleinimid und 4-Aminobenzocyclobuten. Das Addukt kann als Kleber verwendet werden, wobei nach der Aushärtung eine temperaturbeständige Verbindung der verklebten Bauteile erhalten wird.

EP 0 392 354 A1

Xerox Copy Centre

# Härtbare Bismaleinimid-Addukte

Die Erfindung betrifft thermisch härtbare Bismaleinimid-Addukte, die insbesondere als Kleber für hitzebeanspruchte Bauteile geeignet sind.

Zum Kleben von Bauteilen, die erhöhter Temperatur ausgesetzt sind, werden üblicherweise Epoxidharze verwendet. Bei Temperaturen oberhalb von 150 °C sind derartige Verklebungen jedoch nicht mehr stabil, oberhalb von 200 °C fällt die Zugscherfestigkeit stark ab. Ähnliches gilt auch für Kleber auf Basis von einfachen Bismaleinimiden.

Aus US-A 4 675 370 und 4 711 964 sind Addukte der Formeln

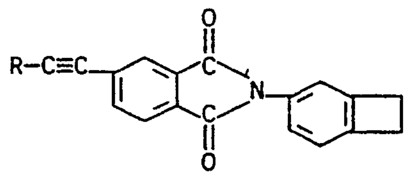

bzw.

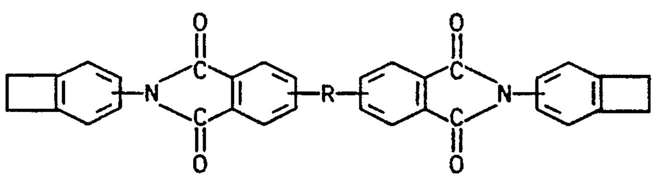

bekannt, die sich als Matrixharze für Faserverbundwerkstoffe und als temperaturbeständige Kleber eignen sollen.

Derartige Kleber haben jedoch eine verhältnismäßig hohe "onset"-Temperatur, d.h., beim Aushärten des Verbunds erfolgt die Polymerisation, welche zur Vernetzung des Klebers führt, erst bei einer sehr hohen Temperatur. Bei dieser Temperatur ist aber die Viskosität des Klebers so niedrig, daß er zwischen den zu verklebenden Oberflächen herausläuft. Der Erfindung lag nun die Aufgabe zugrunde, thermisch härtbare Verbindungen zu entwickeln, die z.B. für temperaturbeständige Verklebungen geeignet sind. Die Kleber sollen insbesondere eine relativ niedrige "onset"-Temperatur aufweisen und die Verklebungen sollen eine über einen großen Temperaturbereich konstant hohe Zugscherfestigkeit haben.

Diese Aufgaben werden durch die erfindungsgemäßen Bismaleinimid-Addukte gelöst.

Gegenstand der Erfindung sind demzufolge thermische härtbare Bismaleinimid-Addukte der allgemeinen Formel

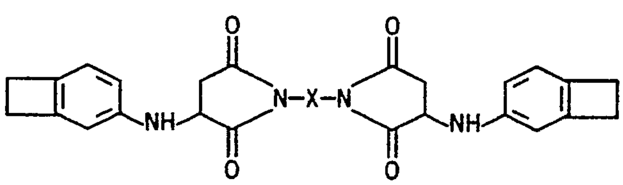

,

worin X eine zweiwertige aromatische oder cycloaliphatische, gegebenenfalls Heteroatome enthaltende Gruppierung ist.

Beispiele für Gruppierungen X sind:

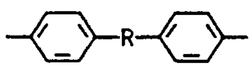 , worin R eine zweiwertige Gruppe, z.B. $SO_2$, CO, O, S, $CH_2$ oder $C(CH_3)_2$ sein kann, ferner

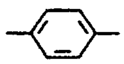 , 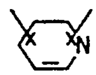 ;

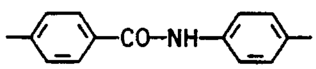

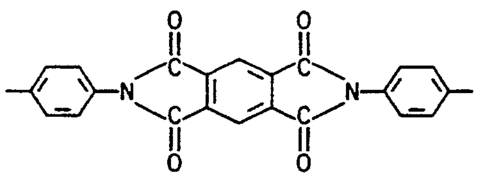

Die Herstellung der erfindungsgemäßen Addukte erfolgt durch Michael-Addition von etwa 2 Mol 4-Aminobenzocyclobuten an 1 Mol Bismaleinimids der Formel

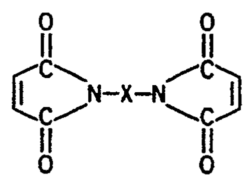 ,

worin X die oben genannte Bedeutung hat.

Falls weniger als 2 Mol 4-Aminobenzocyclobuten eingesetzt werden, können die Imid-Protonen des Addukts sich an überschüssiges Bismaleinimid addieren, so daß das Reaktionsgemisch auch Moleküle folgender Struktur enthalten kann:

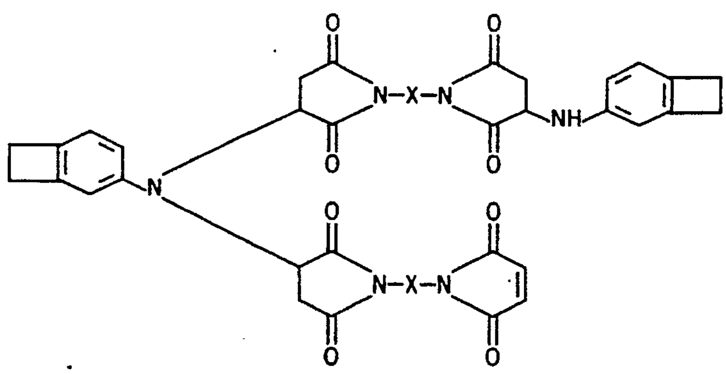

Die erfindungsgemäßen Addukte sind je nach der Art der Gruppierung X bei Raumtemperatur hochviskose bis feste Substanzen. Sie sind im allgemeinen in polaren Lösungsmitteln löslich. Bei Temperaturen oberhalb von 150 °C, vorzugsweise zwischen 170 und 230 °C sind sie härtbar, wobei ein pericyclischer oder radikalischer Mechanismus anzunehmen ist. Die Härtungsprodukte sind unlöslich, unschmelzbar und zersetzen sich erst oberhalb von 350 - 400 °C.

Die erfindungsgemäßen Addukte eignen sich als temperaturbeständige Kleber, als Matrixharze für Faserverbundwerkstoffe und als Gießharze zur Herstellung von elektrotechnischen Bauteilen.

Von besonderem Vorteil ist ihre Verwendung zum Verkleben von Bauteilen, die hohen Temperaturen ausgesetzt sind.

Die Bauteile können aus verschiedenartigen Materialien bestehen, z.B. aus Metall, insbesondere Aluminium oder Stahl, oder aus Kunststoff, vorzugsweise einem Faserverbundwerkstoff.

Die Addukte werden auf die zu verklebende Oberfläche nach üblichem Verfahren aufgebracht, z.B.

durch Aufstreichen, gegebenenfalls bei etwas erhöhter Temperatur, aus Lösung oder in Form eines Films. Dabei kann der erfindungsgemäße Kleber übliche Zusatzstoffe enthalten, wie Füllstoffe, z.B. $CaCO_3$, $SiO_2$ oder Glas; radikalisch polymerisierende Comonomere, z.B. Bismaleinimide oder Acetylengruppen enthaltende Verbindungen; ferner Kunststoffe, wie z.B. temperaturbeständige Phosphazen-Kautschuke oder Thermoplasten, wie Polysulfone oder Polyimide.

Es ist grundsätzlich auch möglich, die Ausgangsstoffe für die erfindungsgemäßen Addukte, d.h. Bismaleinimid und 4-Aminobenzocyclobuten, auf die zu verklebenden Oberflächen aufzubringen, durch Temperaturerhöhung die Bismaleinimid-Addukte in situ herzustellen und in einem Zug auszuhärten.

Beispiele

1. 0,1 Mol 4.4′-Diaminodiphenylmethan-Bismaleinimid werden mit 0,2 Mol 4-Aminobenzocyclobuten unter Stickstoff 4 h bei 120 °C gerührt. Es entsteht ein bei Raumtemperatur hochviskoses Addukt, das bei 220 °C eine Gelzeit von 2 min aufweist. Das Addukt wird mit einem Rakel auf die glatten Oberflächen von Stahlbändern aufgebracht. Die Oberflächen werden unter leichtem Druck zusammengefügt. Anschließend wird 1 h bei 180 °C, dann 5 h bei 220 °C ausgehärtet. Die Zugscherfestigkeit wird in Anlehnung an DIN 53 283 gemessen. Ergebnis s. Tabelle.

2. 0,1 Mol 4.4′-Diaminodiphenylmethan-Bismaleinimid und 0,2 Mol 4-Aminobenzocyclobuten werden bei Raumtemperatur zu einer viskosen Masse vermischt. Diese wird auf Stahloberflächen wie unter 1 beschrieben aufgebracht und anschließend gehärtet. Dabei ist in situ ein Addukt gebildet worden, das bei der anschließenden Härtung vernetzt.

3. Zum Vergleich wurde 4.4-Diaminodiphenylmethan-Bismaleinimid allein auf die Stahloberflächen aufgebracht und gehärtet, wobei ebenfalls eine Polymerisationsreaktion eintritt.

| Ergebnisse | | |
|---|---|---|
| Beispiel | Meßtemperatur | Zugscherfestigkeit |
| 1 | - 55 °C<br>23 °C<br>220 °C | 9 MPa<br>9 MPa<br>8 MPa |
| 2 | - 55 °C<br>23 °C<br>220 °C | 9 MPa<br>9 MPa<br>7 MPa |
| 3 | - 55 °C<br>23 °C<br>220 °C | 10 MPa<br>10 MPa<br>5 MPa |

**Ansprüche**

1. Thermisch härtbare Bismaleinimid-Addukte der allgemeinen Formel

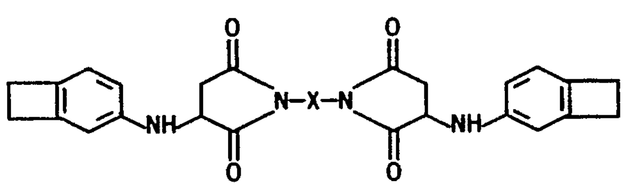

,

worin X eine zweiwertige aromatische oder cycloaliphatische, gegebenenfalls Heteroatome enthaltende Gruppierung ist.

2. Bismaleinimid-Addukte nach Anspruch 1, dadurch gekennzeichnet, daß X eine Gruppierung

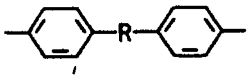 ist,

wobei R $SO_2$, CO, O, S, $CH_2$ oder $C(CH_3)_2$ bedeutet.

3. Verfahren zum Verbinden von Bauteilen, die hohen Temperaturen ausgesetzt sind, durch Aufbringen der Bismaleinimid-Addukte nach Anspruch 1 auf die Oberfläche der Bauteile, Zusammenfügen der Oberflächen und Aushärten der Addukte bei Temperaturen oberhalb von 150 °C.

Europäisches Patentamt

# EUROPÄISCHER RECHERCHENBERICHT

Nummer der Anmeldung

EP 90 10 6519

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | US-A-4 711 964 (L.-S. TAN et al.) * Ansprüche 1-3,7; Spalte 9, Zeilen 35-39; Beispiel 8 * --- | 1,2 | C 07 D 207/416<br>C 09 J 179/08 |
| A,D | US-A-4 675 370 (L.-S. TAN et al.) * Ansprüche 1,11 * --- | 1,2 | |
| A | JOURNAL OF POLYMER SCIENCE, PART A, POLYMER CHEMISTRY Band 26, 1988, Seiten 1819-1834; L.-S. TAN et al.: "Benzocyclobutene in Polymer Synthesis. I. Homopolymerization of Bisbenzocyclobutene Aromatic Imides to Form High-Temperature Resistant Thermosetting Resins" * Seite 1822, Schema 2; Seite 1823, Schema 3; Seite 1824, Tabelle I; Seite 1825, Tabelle II * --- | 1,2 | |
| | | | **RECHERCHIERTE SACHGEBIETE (Int. Cl.5)** |
| A | WO-A-8 705 303 (DOW CHEMICAL CO.) * Ansprüche 1,2,18,19 * --- | 1 | |
| A | US-A-4 795 827 (K.J. BRUZA et al.) * Spalte 13, Beispiel 5 * --- | 1 | C 07 D 207/00<br>C 09 J 179/08 |
| A | DE-B-2 925 261 (T. GOLDSCHMIDT AG) * Ansprüche 1,2; Spalten 5,6 oben, Formelschema * ----- | 3 | |

Der vorliegende Recherchenbericht wurde für alle Patentansprüche erstellt

| Recherchenort | Abschlußdatum der Recherche | Prüfer |
|---|---|---|
| BERLIN | 20-07-1990 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument